# EUROPEAN PATENT APPLICATION

(11) **EP 4 480 510 A1**
(43) Date of publication of application: **25.12.2024**
(21) Application number: 23756591.6
(22) Date of filing: 14.02.2023
(51) Int. Cl.: A61L 27/24, A61L 27/58, A61L 27/22, A61L 27/54, A61F 2/28, A61C 8/02

(54) **BIOABSORBABLE AND PHOTOCURABLE COMPOSITION, BIOABSORBABLE GUIDED TISSUE REGENERATION COMPOSITION, AND GRAFTING METHOD USING SAME**

(30) Priority: 15.02.2022 KR 20220019710
(71) Applicant: Nibec Co., Ltd., Chungcheongbuk-do 27816 (KR); Seoul National University R&DB Foundation, Seoul 08826 (KR)
(72) Inventor: PARK, Yoon Jeong, Seoul 08291 (KR); CHUNG, Chong-Pyoung, Seoul 05618 (KR); SEOL, Yang-Jo, Seoul 04983 (KR); LEE, Jue-Yeon, Gwacheon-si, Gyeonggi-do 13831 (KR); JO, Beom Soo, Seoul 05817 (KR)
(74) Representative: J A Kemp LLP
(86) International application number: PCT/KR2023/002116
(87) International publication number: WO 2023/158179

(57) **Abstract**

A bioabsorbable and photocurable composition, a bioabsorbable guided tissue regeneration composition, and a grafting method using same are provided. The bioabsorbable and photocurable composition includes a photosensitizer and collagen, and is applied onto an in vivo graft site filled with a graft material, so as to cover the graft material while filling an empty space of the graft site, and the photosensitizer is activated by the irradiation of visible light so that the collagen is cross-linked, and the collagen cross-linked by the visible light fills the minute empty space of the graft site and closely binds the graft material to fix the graft material.

## Description

### [Technical Field]

This disclosure relates to a bioabsorbable and photocurable composition, a bioabsorbable guided tissue regeneration composition including the same, and a grafting method using same.

### [Background Art]

In order to treat various bone loss or defects (hereinafter referred to as damage) caused by inflammation, tumor, trauma, and skeletal disease, it is necessary to fill the loss or defect area with a graft material. However, if only the graft material is filled in the damaged area, there is a disadvantage that it is difficult to maintain the graft material at the graft site.

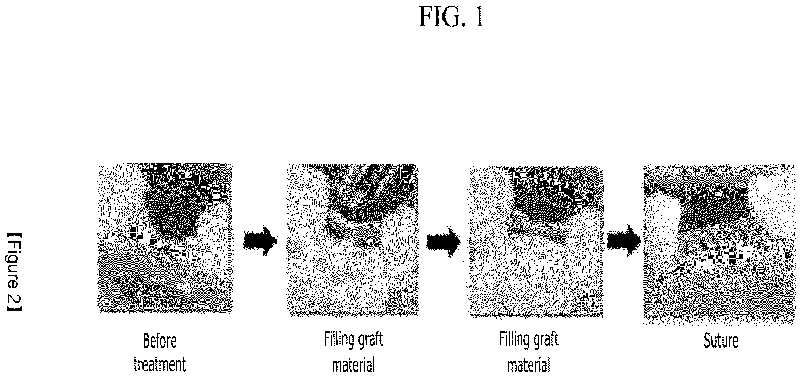

Accordingly, conventionally, as shown in FIG. 1, tissue regeneration is induced by a technique of filling the damaged area with a graft material, covering it with a shielding film, first suturing the shielding film, and then covering the skin with a second suture. However, as shown in FIG. 1, when the graft material and the shielding film are used at the same time, regeneration can be achieved smoothly only when the graft material implanted at the lower portion and the shielding film at the upper portion are in close contact with each other. However, because it is difficult to achieve perfect adhesion between the graft material and the shielding film, a gap occurs between the graft material and the shielding film, resulting in a lower implantation success rate.

Therefore, a new type of material and grafting method that can maintain adhesion while preventing loss of the graft material is required.

### [Disclosure]

### [Technical Problem]

The present disclosure provides a bioabsorbable and photocurable composition that can maintain adhesion while preventing loss of the graft material.

The present disclosure provides a bioabsorbable guided tissue regeneration composition including a bioabsorbable and photocurable composition.

The present disclosure provides a grafting method.

### [Technical Solution]

The bioabsorbable and photocurable composition according to the embodiment includes a photosensitizer and collagen, is applied onto an in vivo graft site filled with a graft material, so as to cover the graft material while filling an empty space of the graft site, and the photosensitizer is activated by the irradiation of visible light so that the collagen is cross-linked, and the collagen fills the fine empty space of the graft site and closely binds the graft material to fix the graft material.

A bioabsorbable guided tissue regeneration composition according to embodiments includes a graft material including a first agent including collagen as a main ingredient and a second agent including a peptide formulated to be immersed in the first agent, and a bioabsorbable and photocurable composition including a photosensitizer and collagen, wherein the bioabsorbable and photocurable composition is applied to an graft site filled with a graft material so as to cover the graft material while filling an empty space of the graft site, and the photosensitizer is activated by the irradiation of visible light so that the collagen is cross-linked, and the collagen cross-linked by the visible light fills the fine empty space of the graft site and closely binds the graft material to fix the graft material.

A grafting method according to embodiments includes filling a vivo graft site with a graft material, applying a bioabsorbable and photocurable composition including a photosensitizer and collagen onto the graft site filled with the graft material, thereby filling the empty space of the graft site and covering the graft material, and irradiating visible light to the bioabsorbable and photocurable composition to activate the photosensitizer and cross-link the collagen, so that the collagen cross-linked by the visible light fills the fine empty space of the graft site and closely binds the graft material to fix the graft material.

Other embodiments of the present invention are included in the following detailed description.

### [Advantageous Effects]

According to the bioabsorbable and photocurable composition and the grafting method using the same according to embodiments, after the bioabsorbable and photocurable composition covers the upper surface of the graft material, fills the remaining empty space of the graft site filled with the graft material, then fills the fine empty space of the graft site, and closely adheres the graft material. Therefore, it is possible to prevent loss of the graft material and maintain close adhesion with the graft material, thereby allowing tissue regeneration to occur effectively.

According to the guided tissue regeneration composition according to the embodiment and the tissue regeneration inducing method using the same, a graft material with anti-inflammatory or antibacterial activity and a bioabsorbable and photocurable composition are used together to exert anti-inflammatory or antibacterial function, it can effectively form the microenvironmental factors necessary for tissue regeneration, while simultaneously preventing loss of the graft material and maintaining close adhering to the graft material, allowing tissue regeneration to occur effectively.

### [Description of the Drawings]

FIG. 1 is a schematic view showing a typical conventional bone grafting procedure.
FIG. 2 is a flowchart showing a grafting method according to an embodiment.
FIG. 3 is a schematic view showing the process in which photocuring occurs in a photocurable composition.
FIG. 4 shows the results of analyzing the purity using liquid chromatography after synthesizing a peptide having the amino acid sequence of SEQ ID NO: 1.
FIG. 5 shows the results of analyzing the molecular weight using mass spectrum after synthesizing a peptide having the amino acid sequence of SEQ ID NO: 1.
FIG. 6 shows the results of enzyme-linked immunosorbent assay (ELISA) for IL-6 and TNF-α.
FIG. 7 shows the results of Western blot analysis of pNFkB and plkBa.
FIG. 8 shows the results of measuring the antibacterial activity of a peptide having the amino acid sequence of SEQ ID NO: 1.
FIG. 9 shows the results of treating a biofilm with a peptide having the amino acid sequence of SEQ ID NO: 1.
FIG. 10 shows the results of analyzing the degree of intracellular penetration of the peptide having the amino acid sequence of SEQ ID NO: 1 using a confocal microscope.
FIG. 11 shows the degree of intracellular penetration of a peptide having the amino acid sequence of SEQ ID NO: 1 into rabbit gingival tissue.
FIG. 12 shows the degree of intracellular penetration of a peptide having the amino acid sequence of SEQ ID NO: 1 into adult dog gingival tissue.
FIG. 13 shows the results of measuring the antibacterial activity of a collagen sponge block including a peptide having the amino acid sequence of SEQ ID NO: 1.
FIGS. 14 and 15 show the results of periodontal tissue regeneration confirmed by radiography by treating an adult dog periodontitis model with a collagen gel including a peptide having the amino acid sequence of SEQ ID NO: 1, and the degree of alveolar bone regeneration is numerically shown. The square represents the alveolar bone regenerated by the graft material.
FIG. 16 is a photograph showing the gel formation state according to the change in collagen content in the photocurable composition.
FIG. 17 is a photograph showing the gel formation state according to the change in riboflavin content in the photocurable composition.
FIG. 18 is a photograph showing the gel formation state according to the change in NaOH content in the photocurable composition.
FIG. 19 is a photograph showing changes in the properties of collagen gel including riboflavin due to light irradiation.
FIG. 20 is a graph showing the results of measuring the viscosity of collagen gel including riboflavin before and after curing.
FIG. 21 is a photograph measuring the stability of collagen gel including riboflavin.
FIG. 22 is a photograph showing the stages of implantation using a photocurable composition (collagen gel including riboflavin) in an adult dog suffering from peri-implantitis.

### [Mode for Invention]

Hereinafter, the embodiments will be described in detail so that those skilled in the art can easily perform the embodiments. The embodiments may be implemented in various different forms, and the present disclosure is not limited only to the specific embodiments described herein.

Unless the definition of some terms used in the present disclosure is defined otherwise below, all technical and scientific terms used herein have the same meaning as commonly understood by one of ordinary skill in the art to which the present invention belongs.

The techniques and processes described in this disclosure are generally performed according to conventional methods, which are presented throughout this application. In general, nomenclatures and experimental procedures in molecular biology, biochemistry, analytical chemistry, and cell culture used in this disclosure are well known in the art and are the same as those commonly used.

The present disclosure relates to a bioabsorbable and photocurable composition and a grafting method using same. The vivo graft site refers to an area including various bone loss or defects (hereinafter referred to as bone damage) caused by inflammation, tumor, trauma, and skeletal disease.

Bone damage areas may include both dental and non-dental damage areas. The most representative area of dental damage is damage caused by periodontal disease. Periodontal disease includes gingivitis limited to the gums, periodontitis that spreads inflammation to the alveolar bone surrounding the tooth roots, and peri-implantitis that is a complication after surgically embedding artificial materials in the jawbone, affecting the tissues around the implant, resulting in loss of supporting bone. Additionally, the dental injury site may include a dislocated trauma tooth site or an extraction socket site.

Non-dental damage areas may include various damage areas, such as congenital damage, damage due to cancer resection, acquired damage due to aging, bone degeneration and osteoporosis, and trauma-related damage such as fractures.

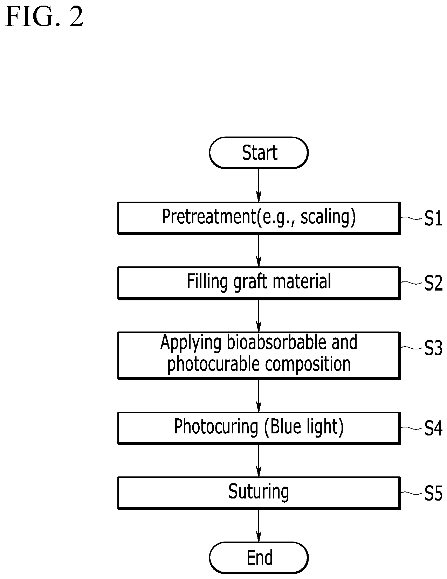

As shown in FIG. 2, a pretreatment process may be performed before filling the graft site with graft material (S1).

For example, when treating periodontal disease, which is typical of dental damage, the process of regenerating periodontal tissue may be performed after scaling and/or root planing to remove plaque and tartar that cause periodontal disease.

Subsequently, the graft material is filled in the graft site (S2).

The graft material can include graft materials from various fields such as a skin graft material, a bone graft material, a musculoskeletal regeneration material, a nerve regeneration material, and a vascular regeneration material. The graft material may basically include various graft matrices such as extracellular matrix protein, bone mineral, chitosan, biodegradable synthetic polymer, etc.

The extracellular matrix protein may be one or more selected from collagen, hyaluronic acid, elastin, chondroitin sulfate, and fibroin. These extracellular matrix proteins can be both derived from humans and animals, and recombinant proteins produced from microorganisms.

Among extracellular matrix proteins, collagen may be the most suitable. Collagen is produced by fibroblasts in the human body and is a main component of connective tissue, which is most commonly found in the body, and is a fibrous protein distributed throughout our body, including bones, skin, joints, membranes of various organs, and hair. In addition, a lot of collagen exists around the axons of central and peripheral nerves. Since collagen hydrogels present in the body have slightly different functions and roles depending on the tissue area, the collagen concentration and degree of orientation of collagen hydrogels in each area are different. For example, bones are characterized by a structure in which micro/nanocrystalline minerals are located between oriented collagen fibers. Collagen is an absorbable material in the body that decomposes easily, so when implanted into the body, it is suitable as a graft matrix because it can gradually decompose and be replaced by regenerated bone. In the case of collagen, it is desirable to use type 1 or type 3 isolated from pig skin.

The bone mineral component may be one or more selected from bio-derived bone mineral powder derived from allogeneic or xenogeneic bone, synthetic hydroxyapatite, and calcium phosphate cement (CPC) powder including tetracalcium phosphate (TTCP), dicalcium phosphate anhydride (DCPA), and monocalcium phosphate monohydrate (MCPM).

The biodegradable synthetic polymer may be any one or more selected from polyglycolide, polylactide, polyglycolide lactide, and polycatrolactone.

The graft matrix may be in a form of a preparation consisting of paste, powder, putty, gel, hydrogel, matrix, granule, particle, lyophilized powder, lyophilized bone, demineralized lyophilized bone, fresh or fresh frozen bone, cortical cancellous bone mix, pellet, strip, plug, membrane, lyophilized powder that is returned to water to form a wet cake, sphere, sponge, block, morcelle, stick, wedge, cement, or amorphous particles.

If necessary, the graft matrix may further include therapeutic agents having anti-inflammatory, antibacterial, or cell-penetrating properties. The therapeutic agent may be a peptide with anti-inflammatory, antibacterial, or cell-penetrating properties.

In one embodiment for treating dental damage, the graft material may exist as a combination of a first agent including collagen as a main ingredient and a second agent including peptides formulated to be immersed in the first agent, which are mixed before implantation. can be used.

The first agent including collagen as a main ingredient may include collagen sponge blocks of various sizes or a collagen sponge block and a bone mineral composite. If collagen powder is used, a three-dimensional structure cannot be formed and an environment in which new tissue can be easily regenerated cannot be provided, so it is preferable to use a collagen sponge block. When treating dental damage, depending on the size of the teeth, such as premolars and molars, collagen sponge blocks with a size of 6X5X7mm or 8×7×9mm, collagen sponge blocks of 6X6X3mm, 6X6X6mm, 7X8X9mm, or 9×10×11mm and a bone mineral composite

The first and second agents can be mixed and used immediately before implantation. When mixed and used immediately before implantation, collagen and peptide can be maintained in a sterile state and has the merit of maintaining high peptide activity. In general, storing proteins or peptides in a lyophilized state allows them to be stored for a long period of time without a decrease in activity compared to storing them in a solution state. Therefore, peptides are stored in a lyophilized state and then used as a collagen sponge just before implantation. This is because the activity of the peptide (anti-inflammatory activity, antibacterial activity, etc.) can be maintained high after implantation by mixing it into the block.

From one viewpoint, the peptide formulated to be capable of immersing the first agent constituting the graft material is a peptide having the amino acid sequence of SEQ ID NO: 1.
SEQ ID NO: 1: GKCSTRGRKSSRRKK

In an aspect, after treatment with the peptide having the amino acid sequence of SEQ ID NO: 1, the intracellular release amount of inflammatory markers IL-6 and TNF-α was measured using enzyme-linked immunosorbent assay (ELISA), and the peptide having the amino acid sequence of SEQ ID NO: 1 exhibited anti-inflammatory activity almost similar to that of IL-4, a cytokine with anti-inflammatory effect. In addition, the inhibitory effect on the expression of pNF-κB, an inflammation-related enzyme, by the peptide having the amino acid sequence of SEQ ID NO: 1 was confirmed by Western blot. As a result, the peptide having the amino acid sequence of SEQ ID NO: 1 inhibits the expression of pNF-κB induced by LPS.

In another aspect, it was confirmed that the peptide having the amino acid sequence of SEQ ID NO: 1 has excellent antibacterial activity against periodontitis-causing bacteria, Aggregatibacter actinomycetemcomitans and Porphyromonas gingivalis, and has equal or similar antibacterial activity compared to minocycline, which is widely used in the treatment of periodontitis

In addition, as a result of treating an artificial biofilm made using periodontitis-causing bacteria with a peptide having the amino acid sequence of SEQ ID NO: 1, it was confirmed that it had antibacterial activity and further cell penetrating ability.

From another viewpoint, the present disclosure relates to a peptide having cell penetrating ability.

In one aspect, it was confirmed through in vitro and in vivo experiments that a peptide having the amino acid sequence of SEQ ID NO: 1 penetrates into cells. Specifically, as a result of treating mouse macrophages (RAW264.7) with a peptide having the amino acid sequence of SEQ ID NO: 1 and confirming whether it penetrates into the cells, it was confirmed that the peptide having the amino acid sequence of SEQ ID NO: 1 penetrated into the cells. In addition, it was confirmed that the peptide having the amino acid sequence of SEQ ID NO: 1 penetrates not only at the cell level but also at the tissue level. When the gingival tissue of a living rabbit and an adult dog were treated with a peptide having the amino acid sequence of SEQ ID NO: 1, it was confirmed that it penetrated into the gingival tissue of a living rabbit and the gingival tissue of an adult dog.

In the case of periodontitis or peri-implantitis, various types of periodontitis-causing bacteria cluster together to form a thick biofilm. In the case of periodontitis or periimplantitis, various types of periodontitis-causing bacteria cluster together to form a thick biofilm. Because these biofilms become increasingly resistant to antibiotics, they are difficult to remove by administering antibiotics alone. Therefore, it is necessary to pass through this biofilm and at the same time have antibacterial activity against periodontitis-causing bacteria that make up the biofilm. Since the peptide having the amino acid sequence of SEQ ID NO: 1 can penetrate into cells on its own, there is no need to use an additional carrier. Therefore, it can penetrate the biofilm formed on teeth to exhibit antibacterial activity or easily penetrate gingival tissue. Furthermore, by using this cell/tissue penetrating ability, the desired anti-inflammatory effect can be increased by chemically conjugating the peptide with an anti-inflammatory function without cell penetrating ability to impart cell penetrating ability.

In addition to the peptide having the amino acid sequence of SEQ ID NO: 1, peptides that can be mixed with collagen may also include peptides of SEQ ID NO: 2 to SEQ ID NO: 5 disclosed in KR1249702, KR1320472, KR1329774, KR1257228, etc., applied and registered by the present applicant, and these specifications are hereby incorporated by reference.
SEQ ID NO 2: CPRRYKQIGTCGLPGTKCCKKP
SEQ ID NO 3: GKCSTRGRKCCRRKK
SEQ ID NO 4: RKIEIVRKKPIFKKATVT
SEQ ID NO 5: CKRKKKGKGLGKKRDPCLRKYK

After dissolving the peptide that is the second agent in physiological saline, the peptide solution is placed in a tube for injection at the graft site, the first agent including collagen as a main ingredient is immersed in it, and then filled into the graft site.

Based on a total of 100 parts by weight of the graft material, 10⁻⁴ to 0.5 parts by weight, and more preferably 10⁻³ to 10⁻¹ parts by weight of the peptide may be included. This is because if the peptide is included in less than 10⁻³ parts by weight, the regenerative effect is not significant, and if it is contained in more than 0.5 parts by weight, it is difficult to include it in a graft material.

Next, the bioabsorbable and photocurable composition is applied to the upper portion of the vivo graft site filled with the graft material (S3).

The bioabsorbable and photocurable composition includes collagen, a photosensitizer, a pH controlling agent, and an isotonic agent. The bioabsorbable and photocurable composition is applied in a gel state when applied to the upper portion of the graft site. Therefore, the viscosity of the bioabsorbable and photocurable composition may have a viscosity that is easy to administer to the graft site and does not flow down. A viscosity of the bioabsorbable and photocurable composition may range from 25 to 270 cP. Therefore, the bioabsorbable and photocurable composition can cover the graft material while efficiently filling the empty space of the graft site filled with the graft material.

Collagen may be a type 1, 2, 3 or 4 collagen solution. Among them, type 1 collagen can be effectively applied. The type 1 collagen is the most connective tissue-derived collagen and is less likely to cause negative immune reactions with the tissue at the graft site. Collagen may be included in the range of 0.75 to 3 w/v%. If it is less than 0.75 w/v% or more than 3 w/v%, gel formation may not be sufficient.

The photosensitizer may be a substance that can induce gelation of collagen by irradiation of light. For example, riboflavin may be a suitable photosensitizer. The riboflavin may be included in the range of 0.0008 w/v% to 0.008 w/v%. Outside this range, gel formation with appropriate viscosity may not be achieved. Preferably, the riboflavin may be included in the range of 0.0008 w/v% to 0.004 w/v%.

Accordingly, a weight ratio of collagen : riboflavin may be 1:0.00056 to 1:0.0533, and within this range, a gel in a desired state can be formed.

NaOH, etc. may be used as a pH controlling agent. The pH controlling agent may be included in the range of 26 mM to 30 mM. Outside this range, gel formation with appropriate viscosity may not be achieved.

As an isotonic agent, a combination of NaCl, KCl, Na₂HPO₄, and KH₂PO₄ can be used.

Next, photocuring is performed (S4).

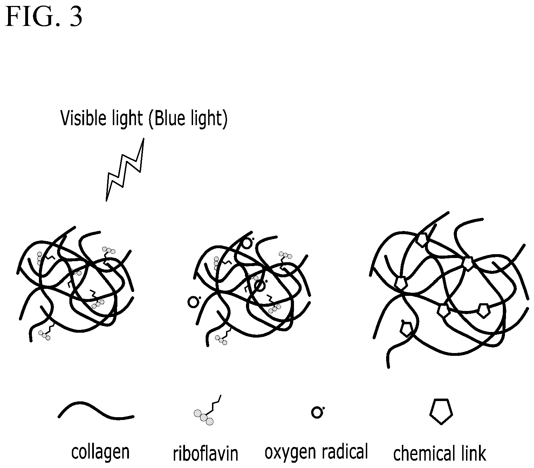

As illustrated in FIG. 3, when visible light is irradiated, riboflavin, a photosensitizer, is stimulated to cause a chemical reaction, and oxygen radicals separated from this form chemical bonding points on collagen, hardening the collagen.

An LED light source in the blue wavelength range is suitable as a visible light source. Although UV photocuring is possible, it can cause damage to cells, so that a light source in the blue wavelength range is appropriate. The light source can irradiate a light source with a wavelength of 450 nm at an output and time sufficient to cure collagen. For example, the output can be up to 2W/cm², and by irradiating the graft site for 20 to 60 seconds so that the patient does not feel heat, the photocurable composition can be sufficiently cured to function as a shield without flowing.

If necessary, the LED light irradiator can be penetrated into the bioabsorbable and photosensitive composition to deliver the light source more effectively so that photocuring occurs evenly throughout the bioabsorbable and photosensitive composition. For example, in the case of periodontitis treatment, an LED light irradiator can penetrate into the photocurable composition filled between the teeth and gums so that the light spreads evenly into the photocurable composition, allowing photocuring to occur more easily.

As a photocurable composition (e.g., collagen gel including a photosensitizer) is cross-linked by visible light, the collagen cross-linked by visible light fills the minute empty space at the graft site and adheres closely to the graft material, allowing the graft material to be fixed. Additionally, collagen gel can also combine with natural collagen present in the graft site. Therefore, regardless of the various shapes and sizes of the graft site, the graft material can be fixed by filling the minute empty space of the graft site and bonding with strong adhesive force. Therefore, problems that arise when applying a conventional shielding film can be solved, and there is no need for a cumbersome process of sealing the shielding film. When initially applied, the viscosity of the photocurable composition increases from 25 cP to 270 cP to 110 cP to 410 cP by 1.5 to 4.5 times by photocuring, allowing the composition to be closely bound to the underlying graft material and prevent the graft material from coming off.

In particular, when the graft material is made of collagen blocks and peptides with antibacterial activity or anti-inflammatory activity, not only does the bonding between the collagen block and the collagen sol enhance adhesive force, but it can also be expected that they act as a scaffold that allows peptides with antibacterial or anti-inflammatory functions to act in a sustained manner. In the case of ointments including minocycline as a main ingredient, which are sold as conventional periodontitis treatments, since minocycline mainly has an antibacterial effect, it is insufficient in inducing regeneration of the graft site accompanied by inflammation. However, when a peptide that exhibits both antibacterial and anti-inflammatory properties is applied, it has the advantage of effectively inducing regeneration of the graft site.

In particular, in the case of periodontal disease, it can reduce inflammation such as various perioritis or periodontitis along with antibacterial activity against periodontitis-causing bacteria, thereby creating an environment suitable for regeneration. In addition, collagen supports natural bone until it is sufficiently regenerated, and over time, it can provide space where it can be decomposed into absorbents and replaced by regenerated bone, thereby increasing the efficiency of periodontal tissue regeneration.

Hereinafter, preferred experimental examples are presented to aid understanding of the present invention. However, the following experimental examples are merely illustrative of the present invention and the scope of the present invention is not limited to the following experimental examples.

### Peptide synthesis

The peptide of SEQ ID NO: 1 was synthesized from the C terminus using a synthesis device using the F-moc solid phase chemical synthesis method. That is, it was synthesized using Rink resin (0.075 mmol/g, 100 to 200 mesh, 1% DVB crosslinking) with Fmoc-(9-Fluorenylmethoxycarbonyl) bound as a blocking group, and after adding 50 mg of Rink Amide MBHA resin to the synthesizer, the resin was swelled with DMF, and then a 20% piperidine/DMF solution was used to remove the Fmoc-group. In order from the C terminus, 0.5 M amino acid solution (solvent: DMF), 1.0 M DIPEA (solvent: DMF&NMP), and 0.5M HBTU (solvent: DMF) were added in 5, 10, and 5 equivalents, respectively and reacted for 1 to 2 hours under a nitrogen stream. After each deprotection and coupling step, washing was performed twice with DMF and methanol. Even after coupling the last amino acid, deprotection was performed to remove the Fmoc-group.

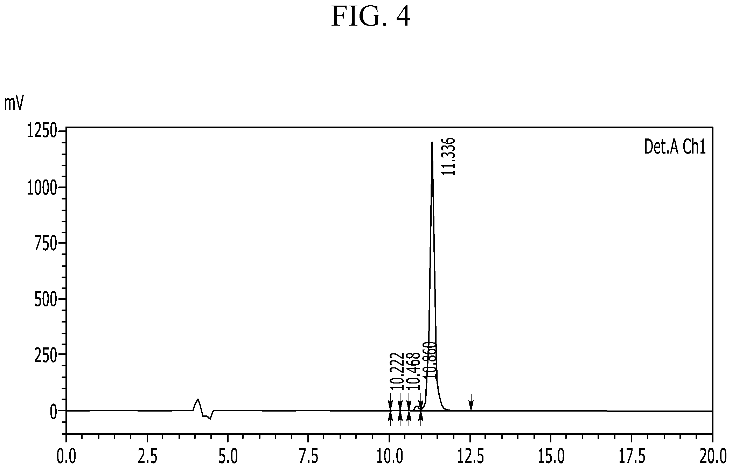

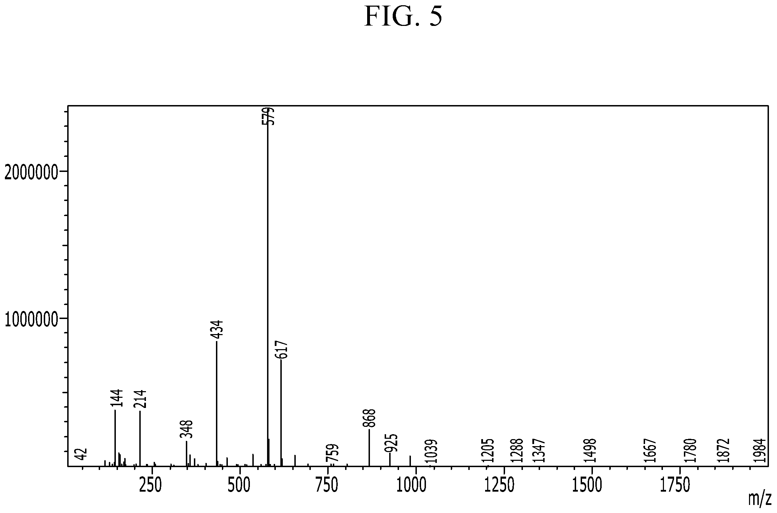

The ninhydrin test method was used to confirm the synthesis. After the test and the completed resin was dried, Reagent K cleavage cocktail was added at a ratio of 20 ml per 1g of resin, and shaken for 3 hours, and the cocktail including dissolved resin and peptide was separated through filtering. Cold ether was added to the filtered solution to crystallize the peptide into a solid phase, which was separated by centrifugation. At this time, the Reagent K cleavage cocktail was completely removed through several washings with ether and centrifugation. The crude obtained in this way was dissolved in distilled water and separated and purified using liquid chromatography. The purified peptide was lyophilized. The results of analyzing the purity and measuring the mass spectrum by liquid chromatography are illustrated in FIGS. 4 and 5.

Referring to FIGS. 4 and 5, it can be confirmed that the synthesized peptide has a purity of 98.2%, a molecular weight of 1733.99, and a 15-mer amino acid sequence.

### Anti-inflammatory Activity Evaluation

### 1) Measurement of production of inflammatory cytokines (IL-6 and TNF-α)

Anti-inflammatory activity was verified using human monocyte cell lines (THP-1, ATCC, TIB-202). In the case of monocyte cell lines, PMA (Phorbol 12-myristate 13-acetate, Merck, P1585) was inoculated to differentiate monocytes into macrophages, and then treated with 1 µg/ml LPS (lipopolysaccharide, Merck, L3024), an inflammation inducer. 16 hours later, 200 µM of the peptide having the amino acid of SEQ ID NO: 1 and 20 ng/ml of IL-4 (R&D system, 204-IL-010/CF) as a positive control were inoculated, and 24 hours later, the culture medium of the cells was obtained. The amount of pro-inflammatory cytokines IL-6 (R&D system, D6050) and TNF-α secreted by cells present in the medium was confirmed through ELISA (enzyme-linked immunosorbent assay).

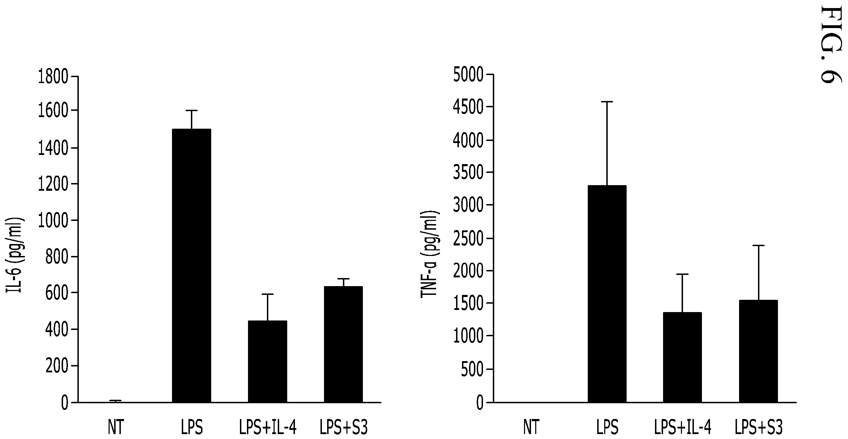

As a result, as shown in FIG. 6, when treated with the peptide having the amino acid sequence of SEQ ID NO: 1 (LPS+S3), it was confirmed that IL-6 and TNF-α values were lowered to a similar level as when treated with IL-4. When treated with LPS alone, IL-6 and TNF-α values increased. Therefore, it was found that the peptide having the amino acid sequence of SEQ ID NO: 1 has an anti-inflammatory effect.

### 2) Western blot of pNFkB and plkBa

Raw264.7 cells were purchased from ATCC (American Type Culture Collection) and used in the experiment. Raw264.7 cells were cultured in a CO₂ incubator at 37 °C supplied with 5% CO₂ and 95% air using DMEM (Dulbecco's modified Eagle medium) medium supplemented with 10% FBS and 100 units/ml antibiotics (penicillin-streptomycin).

Raw264.7 cells were treated with 200 µM of the peptide having the amino acid sequence of SEQ ID NO: 1 for 30 minutes, and then treated with 1 µg/ml LPS (lipopolysaccharide, Merck, L3024). After 8 hours, the treated cells were collected, protease inhibitor cocktail and phosphatase inhibitor cocktail were added to Radioimmunoprecipitation assay buffer (RIPA buffer solution, Thermo Fisher, 89900), and the cells were treated to lyse the cells. After centrifuging the cell lysate and collecting the supernatant, the concentration of the cell lysate was measured using a bicinchoninic acid assay (BCA assay, Thermo Fisher, 23227), and RIPA buffer solution was added thereto to prepare the cell lysate to a final concentration of 5 mg/mL. Then, it was mixed with a buffer solution including 1M Tris-HCl (pH 6.8), 50% glycerol, and 10% SDS in a 4:1 ratio, and then sodium dodecyl sulfate-poly acrylamide gel electrophoresis (SDS-PAGE) was performed. Then, the polyacrylamide gel was transferred to a nitrocellulose membrane, the membrane was blocked with a blocking solution (5% skim milk in Tris-buffered saline with 0.1% Tween^{®}20), and then primary antibodies (phosphorylated NF-κB (Signaling Technology, 3033), NF-κB (Cell Signaling Technology, 8242), phosphorylated IκBα (Cell Signaling Technology, 9246), IκBα (Cell Signaling Technology, 4812) and β-actin (Santa Cruz Biotechnology, sc-47778)) were reacted with the blocking solution at a 1:1000 ratio in the refrigerator overnight. Then, the secondary antibody for each primary antibody was reacted with blocking solution 1:2000 at room temperature for 1 hour, then reacted with ECL (enhanced chemiluminescence, Thermo Fisher, 34094) solution, and then film was developed using ChemiDoc Imaging Systems (Bio-Rad). Phosphorylated NF-κB and phosphorylated plkBa are indicators that can confirm a degree of inflammatory response at the cellular level. The higher the inflammatory response, the more phosphorylated NF-κB and phosphorylated plkBa expression can be confirmed.

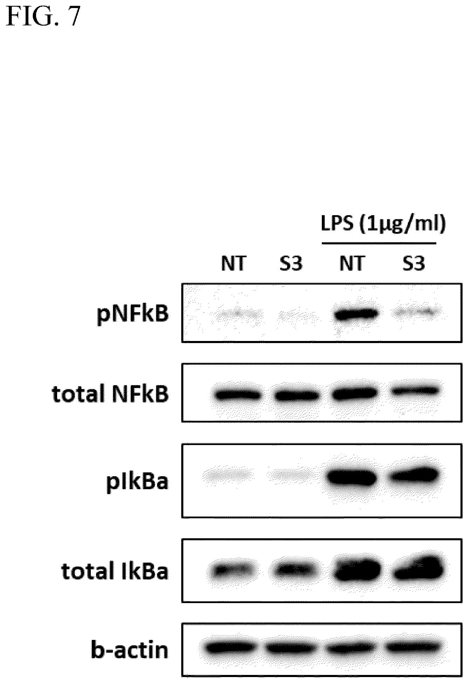

As a result, as shown in FIG. 7, when only LPS was treated, the expression of pNFκB and plkBa increased, but it was confirmed that when treated with LPS after treatment with the peptide having the amino acid sequence of SEQ ID NO: 1, the expression of pNFκB and plkBa was suppressed. Therefore, it was found that the peptide having the amino acid sequence of SEQ ID NO: 1 has an anti-inflammatory effect.

However, when only the peptide having the amino acid sequence of SEQ ID NO: 1 was treated, there was no effect on the expression of pNFκB and plkBa in cells, which indirectly proves that the peptide is safe.

### Antibacterial activity evaluation

### 1) Inhibiting growth of periodontitis-causing bacteria

To evaluate the antibacterial activity, representative periodontitis-causing bacteria, Aggregatibacter actinomycetemcomitans and Porphyromonas gingivalis, were used. Each bacterium was inoculated into BHI (brain heart infusion) liquid medium and cultured in an anaerobic incubator at 37 °C and 120 rpm for 48 hours. After incubation, the peptide having the amino acid sequence of SEQ ID NO: 1 was treated at 100 µM, 250 µM, and 500 µM. As a positive control, minocycline (Merck, M9511) was inoculated at 500 ng/ml, and then 24 hours later, the absorbance was measured at 600 nm using a spectrophotometer to confirm a degree of bacterial growth.

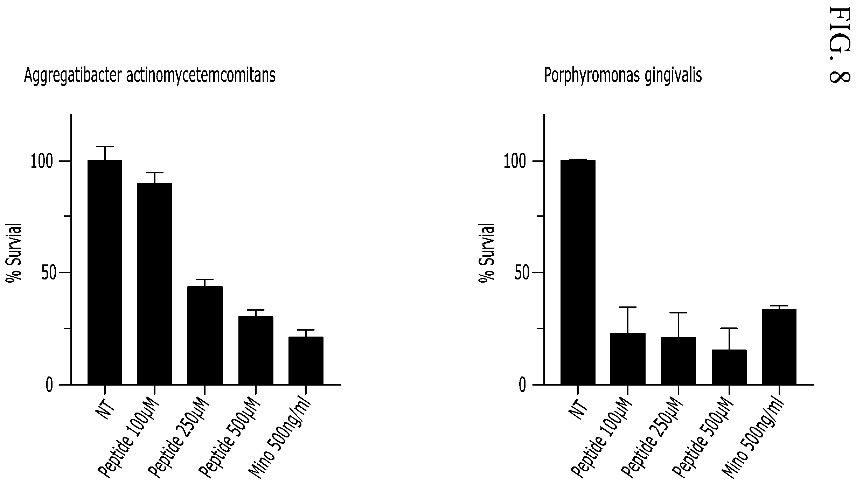

As a result, as shown in FIG. 8, when treated with a peptide having the amino acid sequence of SEQ ID NO: 1, the survival rate of bacteria was less than or equal to 50% for Aa (Aggregatibacter actinomycetemcomitans) at 250 µM or more, and the survival rate of bacteria was found to be less than or equal to 50% for Pg (Porphyromonas gingivalis) at 100 µM or more.

Meanwhile, when compared to the effect of minocycline, when treated with Aa at a concentration of 500 µM, the survival rate was higher than when treated with minocycline 500 ng/ml, but showed a similar effect. The minocycline only has antibacterial activity, whereas the peptide having the amino acid sequence of SEQ ID NO: 1 has both antibacterial activity and anti-inflammatory activity, so that even if there is a slight difference in antibacterial activity, this can be compensated for. For Pg, it showed a better effect than minocycline in all concentration ranges.

### 2) Evaluation of antibacterial activity in biofilm

Periodontitis-causing bacteria exist in the oral cavity in a form of a biofilm, and thus in order to imitate this, an artificial biofilm was created on the surface of a titanium disk using A. a. (Aggregatibacter actinomycetemcomitans) and P. g. (Porphyromonas gingivalis) bacteria. Saliva and bacteria were mixed on a titanium disk, placed in BHI liquid medium, and cultured in an anaerobic incubator at 37 °C. After 72 hours, 50 µg/ml (=29 µM), 100 µg/ml (=58 µM), 250 µg/ml (=144 µM), and 500 µg/ml (=288 µM) peptides and 1 µg/ml minocycline as a positive control were inoculated. 24 hours later after inoculation, a titanium disk with biofilm formed on the surface was obtained and the biofilm was stained using the Filmtracer LIVE/DEAD Biofilm Viability Kit (Thermo Fisher, L10316). Live bacteria are stained green, and dead bacteria are stained red. The titanium disk was observed using a confocal scanning fluorescence microscope.

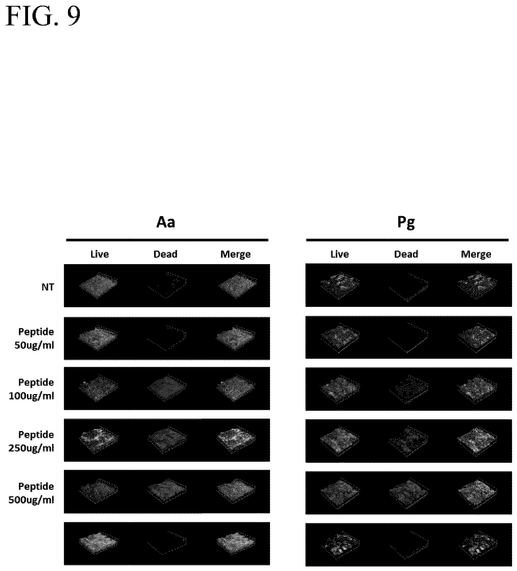

As a result, as shown in FIG. 9, minocycline used as a positive control was unable to penetrate the biofilm and kill the bacteria, so that almost no killed bacteria (red) were observed. On the other hand, when treated with a peptide having the amino acid sequence of SEQ ID NO: 1, killed bacteria (red) were observed starting at a concentration of 50 µg/ml (=29 µM). Therefore, in the case of bacteria existing in the form of a biofilm, existing antibiotics cannot kill them, but the peptide having the amino acid of SEQ ID NO: 1 penetrates the biofilm and has antibacterial activity.

### Cell/tissue penetrating ability evaluation

### 1) Evaluation of cell penetration ability at the cellular level

The N-terminus of the peptide having the amino acid of SEQ ID NO: 1 prepared in Example 1 was fluorescently labeled with Rhodamine B (Tokyo chemical industry Co., LTD., A5102). Raw264.7 cells were distributed 3×10⁴ each into a 4-well chamber and cultured in DMEM medium for 24 hours to stabilize the cells. After 24 hours, fluorescently labeled peptide was added to the medium at concentrations of 50, 100, and 200 µM. After 1 hour of peptide treatment, a degree of penetration of the peptide into cells was observed using a confocal scanning fluorescence microscope (model name, company name). Cell nuclei were stained using 4',6-diamidino-2-phenylindole (DAPI, Thermo Fisher, D1306). DIC represents the case observed under a general microscope, Nuceli represents the nucleus of a cell, Peptide represents the degree of penetration of a peptide bound to rhodamine B, and MERGE represents an image that overlaps images of DIC, Nuclei, and the penetrated peptide.

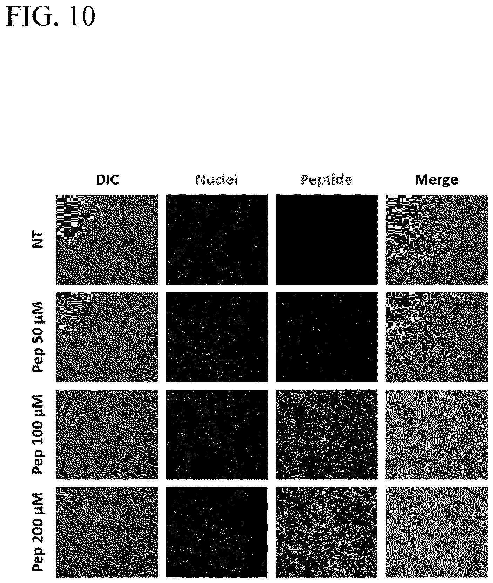

As a result, as shown in FIG. 10, fluorescence of rhodamine B (red) could be confirmed in cells treated with the peptide having the amino acid of SEQ ID NO: 1, but red color could not be confirmed in the untreated experimental group. In addition, it was found that as the concentration of peptide increased, the intensity of fluorescence also increased.

### 2) Evaluation of tissue penetration ability in animal tissue

The amine group of the peptide having the amino acid of SEQ ID NO: 1 was labeled with fluorescence using Alexa Fluor^{™} 680 NHS Ester (Thermo Fisher, A20008). The fluorescent labeling method followed the method provided by the manufacturer. The fluorescently labeled peptide was dissolved in water for injection at a concentration of 250 mg/ml, and 10 µL was injected into the gingival sulcus of the premolars of an adult dog (beagle dog) and the anterior gingival sulcus of a rabbit. After 2 hours, to check the permeability of the peptide in the tissue, the animal was sacrificed, the gingival tissue was collected, and tissues were embedded using Tissue-Tek^{®} O.C.T. Compound (Sakura Finetek, 4583). After sectioning the embedded tissue at 10 µm thickness, fluorescently labeled peptides were confirmed using a confocal scanning fluorescence microscope. The nuclei of tissue cells were stained using DAPI.

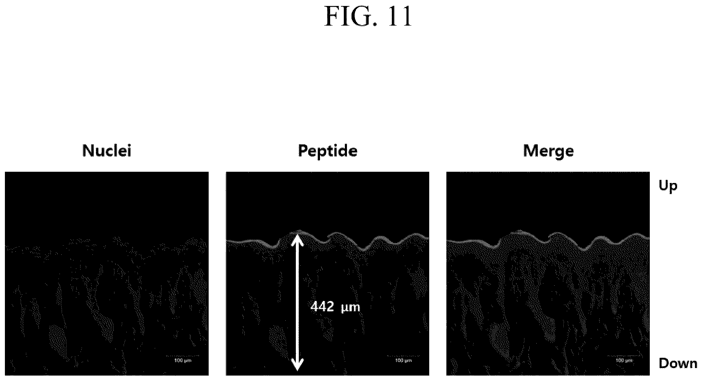

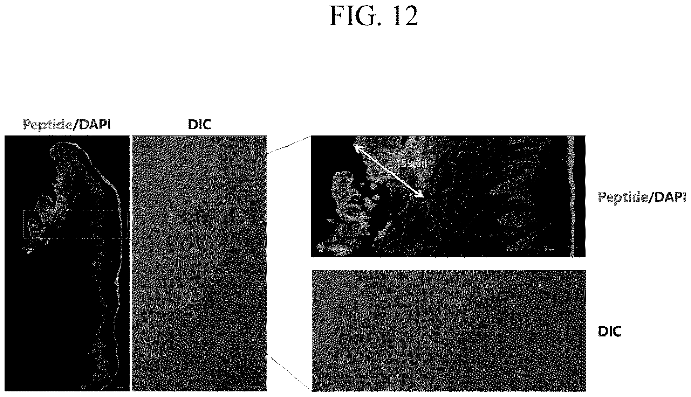

As a result, it was confirmed that the fluorescently labeled peptide penetrated the gingival tissue cells and reached about 500 (440 to 459) µm from the results of the gingival tissue of a rabbit in FIG. 11 and the gingival tissue of an adult dog in FIG. 12.

Therefore, it was confirmed that the peptide having the amino acid sequence of SEQ ID NO: 1 penetrates not only the cell level but also tissue.

### Evaluation of antibacterial activity of graft materials

500 µg/0.5 ml of the peptide having the amino acid of SEQ ID NO: 1 was immersed in the collagen sponge block with a size of 6X5X7mm, left at 4 °C for 8 hours, and then lyophilized. As a positive control, 250 ng/0.5ml of minocycline (Merck, M9511) was immersed in a collagen sponge block with a size of 6X5X7mm, left at 4 °C for 8 hours, and then lyophilized. In order to evaluate the antibacterial activity of a graft material including the peptide having the amino acid of SEQ ID NO: 1, representative periodontitis-causing bacteria, Aggregatibacter actinomycetemcomitans and Porphyromonas gingivalis, were used. Each bacterium was inoculated into BHI (brain heart infusion) liquid medium and cultured in an anaerobic incubator at 37 °C and 120 rpm for 48 hours. After culturing, a collagen sponge block including the peptide having the amino acid sequence of SEQ ID NO: 1 of Example 2 was added to the medium. After 24 hours, the absorbance was measured at a wavelength of 600 nm to confirm a degree of bacterial growth.

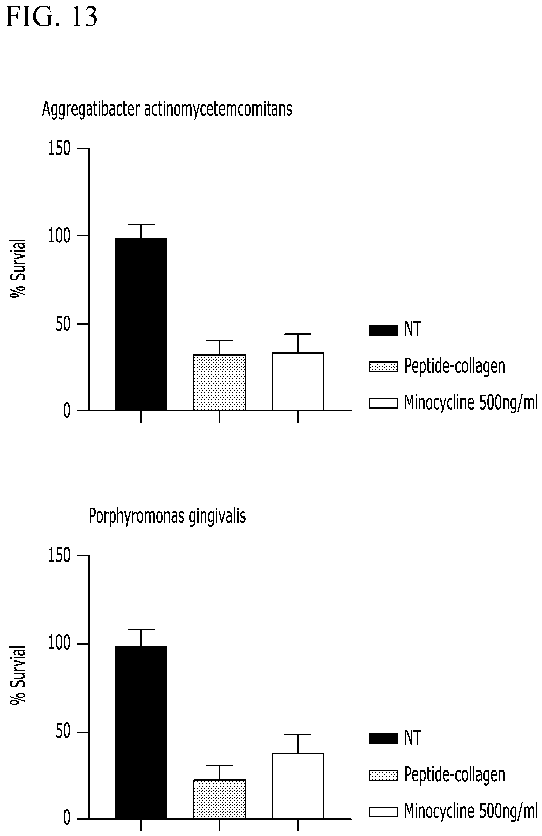

As a result, as shown in FIG. 13, when the collagen sponge block including the peptide having the amino acid sequence of SEQ ID NO: 1 was treated, the survival rate of Aa (Aggregatibacter actinomycetemcomitans) and Pg (Porphyromonas gingivalis) was less than or equal to 40%. Therefore, it was proven that an antibacterial effect exists even when the peptide having the amino acid sequence of SEQ ID NO: 1 was applied to the collagen sponge block.

### Evaluation of periodontal tissue regeneration ability of graft material

Collagen gel was dissolved in 0.1 M PBS at a concentration of 4%. 125 mg of a peptide having the amino acid of SEQ ID NO: 1 was dissolved in 1 g of collagen gel. As a positive control, 20 mg of minocycline was dissolved in 1 g of the same collagen gel.

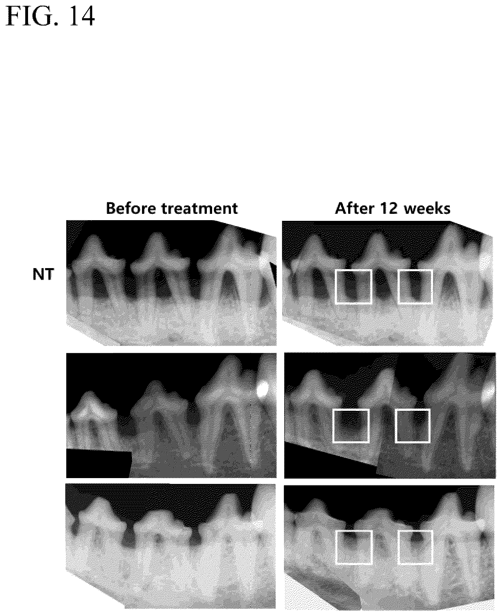

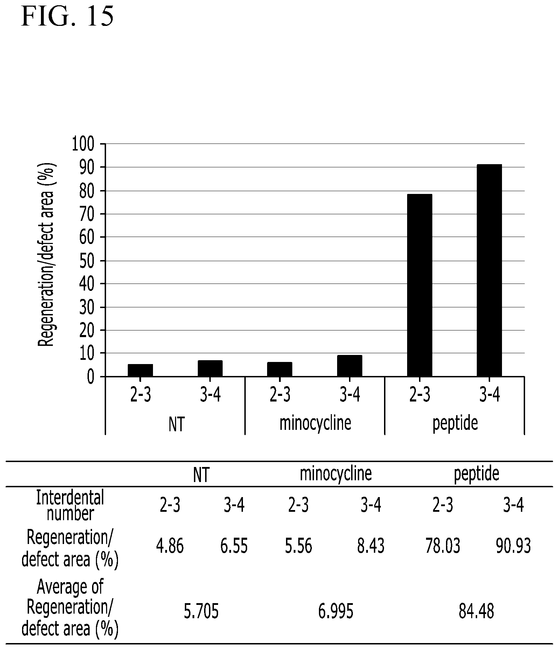

To evaluate the periodontal tissue regeneration ability of a graft material including a peptide having the amino acid of SEQ ID NO: 1, a collagen sponge block including the peptide was implanted after inducing periodontitis in a beagle dog.. Periodontitis was induced by wrapping multiple layers of dental wire around the cervical region of the 2nd, 3rd, and 4th mandibular premolars. Three months after wrapping the wire, the wire was removed, and scaling and root planning were performed. 15 µl of collagen gel including the peptide having the amino acid of SEQ ID NO: 1 and collagen gel including minocycline were each applied to the gingival sulcus twice a week for 5 weeks. Periodontal condition was observed for 12 weeks, radiography (X-ray) was taken, and alveolar bone regeneration was observed. As a result, as shown in FIGS. 14 and 15, the group to which nothing was applied (NT) did not produce alveolar bone after 12 weeks. The group implanted with collagen gel including minocycline showed a similar degree of alveolar bone regeneration as the NT group. However, when the collagen gel applied with the peptide having the amino acid of SEQ ID NO: 1 was implanted, the regeneration of the alveolar bone (white part in the square) increased by more than 10 times compared to the group implanted with the collagen gel including minocycline. Therefore, the collagen gel to which the peptide having the amino acid of SEQ ID NO: 1 was applied was proven to have an excellent periodontal tissue regeneration effect.

### Measurement of gel formation according to changes in collagen content in photocurable compositions

A collagen gel including riboflavin (riboflavin sodium phosphate, Sigma, 77623-50G-F) was prepared using pig-derived type 1 collagen. As shown in Table 1, the gel was prepared by changing the concentration of collagen to 0.375 w/v%, 0.75 w/v%, 1.125 w/v%, 1.5 w/v%, and 3 w/v%, and riboflavin and NaCl, KCl, Na₂HPO₄, KH₂PO₄, and NaOH were fixed to prepare collagen gel. Purified water was used as the solvent.

**(Table 1)**

| Composition of changing the concentration of collagen solution | | | | | | | | |
|---|---|---|---|---|---|---|---|---|
| | Final content | | | | | | | |
| Function | Main ingredie nt | Photose nsitizer | Collage n dissoluti on | pH controlli ng agent | Isotonic agent | | | |
| Composit ion number | Collage n (w/v %) | Riboflav in (w/v %) | Acetic acid (mM) | NaOH (mM) | NaCl (mM) | KCl (mM) | Na₂HPO₄ (mM) | KH₂PO₄ (mM) |
| 1 | 0.375 | 0.0016 | 15 | 26 | 114 | 2.25 | 8.33 | 1.5 |
| 2 | 0.75 | 0.0016 | 15 | 26 | 114 | 2.25 | 8.33 | 1.5 |
| 3 | 1.125 | 0.0016 | 15 | 26 | 114 | 2.25 | 8.33 | 1.5 |
| 4 | 1.5 | 0.0016 | 15 | 26 | 114 | 2.25 | 8.33 | 1.5 |
| 5 | 3 | 0.0016 | 15 | 26 | 114 | 2.25 | 8.33 | 1.5 |

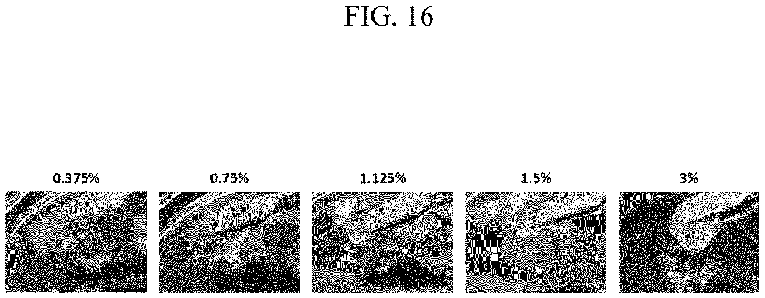

As a result, as shown in FIG. 16, it was confirmed that a gel was formed starting from 0.75% collagen, and the hardest gel was formed at 3%.

### Measurement of gel formation according to changes in riboflavin content in photocurable compositions

As shown in Table 2, the gel was prepared by changing the concentration of riboflavin to 0.0008 w/v %, 0.0016 w/v %, 0.004 w/v %, and 0.008 w/v %, and collagen gel was prepared by fixing collagen, NaCl, KCl, Na₂HPO₄, KH₂PO₄, and NaOH. Purified water was used as the solvent.

**(Table 2)**

| Composition of varying concentration of riboflavin solution | | | | | | | | |
|---|---|---|---|---|---|---|---|---|
| | Final content | | | | | | | |
| Functio n | Main ingredie nt | Photose nsitizer | Collage n dissoluti on | pH controlli ng agent | Isotonic agent | | | |
| Compos ition number | Collage n (w/v %) | Riboflavi n (w/v %) | Acetic acid (mM) | NaOH (mM) | NaCl (mM) | KCl (mM) | Na₂HPO₄ (mM) | KH₂PO₄ (mM) |
| 1 | 1.5 | 0.0008 | 15 | 26 | 114 | 2.25 | 8.33 | 1.5 |
| 2 | 1.5 | 0.0016 | 15 | 26 | 114 | 2.25 | 8.33 | 1.5 |
| 3 | 1.5 | 0.004 | 15 | 26 | 114 | 2.25 | 8.33 | 1.5 |
| 4 | 1.5 | 0.008 | 15 | 26 | 114 | 2.25 | 8.33 | 1.5 |

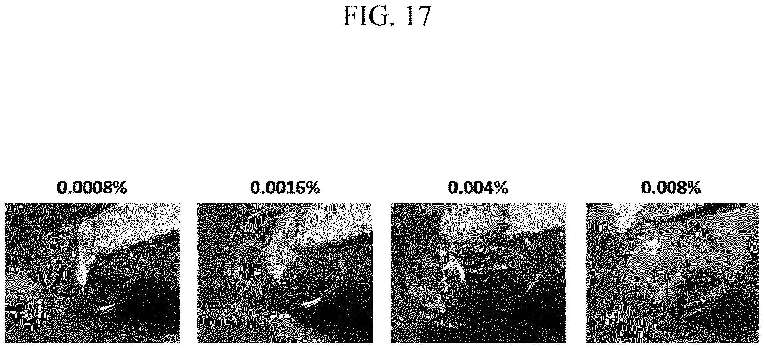

As a result, as shown in FIG. 17, a gel with an appropriate viscosity was formed in the range of 0.0008 w/v% to 0.008 w/v%, and a gel with a more desirable viscosity in the range of 0.0008 w/v% to 0.004 w/v% was formed.

### Measurement of gel formation according to change in NaOH content in photocurable composition

As shown in Table 3, a gel was prepared by changing the NaOH concentration to 20 mM, 26 mM, and 30 mM, and collagen, riboflavin, NaCl, KCl, Na₂HPO₄, and KH₂PO₄ were fixed to prepare a collagen gel. Purified water was used as the solvent.

**(Table 3)**

| Composition with varying pH regulator concentration | | | | | | | | |
|---|---|---|---|---|---|---|---|---|
| | Final content | | | | | | | |
| Functio n | | Photose nsitizer | Collage n dissoluti on | pH controlli ng agent | Isotonic agent | | | |
| Compos ition number | Collage n (w/v %) | Riboflavi n (w/v %) | Acetic acid (mM) | NaOH (mM) | NaCl (mM) | KCl (mM) | Na₂HPO₄ (mM) | KH₂PO₄ (mM) |
| 1 | 1.5 | 0.0016 | 15 | 20 | 114 | 2.25 | 8.33 | 1.5 |
| 2 | 1.5 | 0.0016 | 15 | 26 | 114 | 2.25 | 8.33 | 1.5 |
| 3 | 1.5 | 0.0016 | 15 | 30 | 114 | 2.25 | 8.33 | 1.5 |

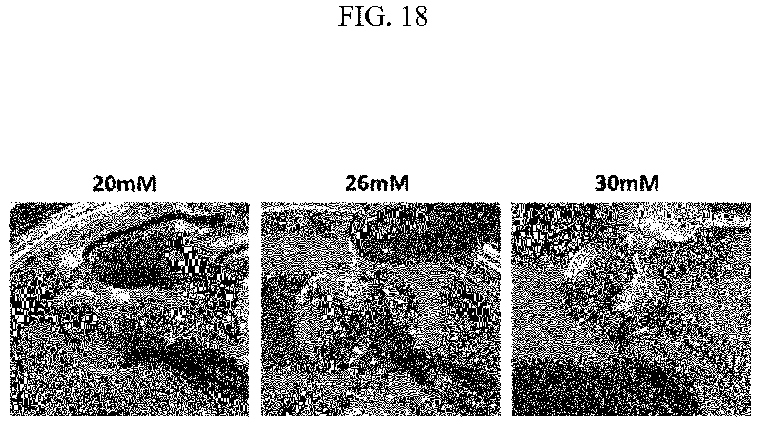

As a result, as shown in FIG. 18, no gel was formed at 20 mM NaOH, but gels were formed at 26 mM and 30 mM NaOH.

### Change in phase of photocurable composition (collagen gel including riboflavin) due to light irradiation

300 µl of collagen gel of composition number 4 in Table 1 was irradiated with light with a wavelength of 450 nm at an intensity of 2 2 W/cm² for 10 seconds, 20 seconds, 30 seconds, 50 seconds, and 60 seconds, and the shape of the collagen gel was observed.

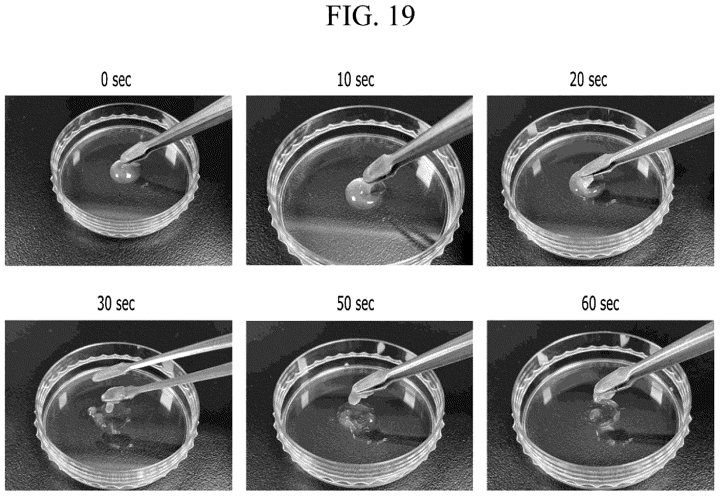

The results are illustrated in FIG. 19. Referring to FIG. 19, no phase change occurred when light was irradiated for 10 seconds. When light was irradiated for 20 seconds, curing progressed and began to change into a solid state, and after 30 seconds, it changed to a solid state.

### Viscosity measurement of photocurable compositions

Gels with composition numbers 2 to 5 listed in Table 1 were measured using a viscometer (DV-II Pro viscosity meter, Brookfiled). During the measurement, spindle number 42 was used, and the viscosity was measured before and after curing at 90 rpm.

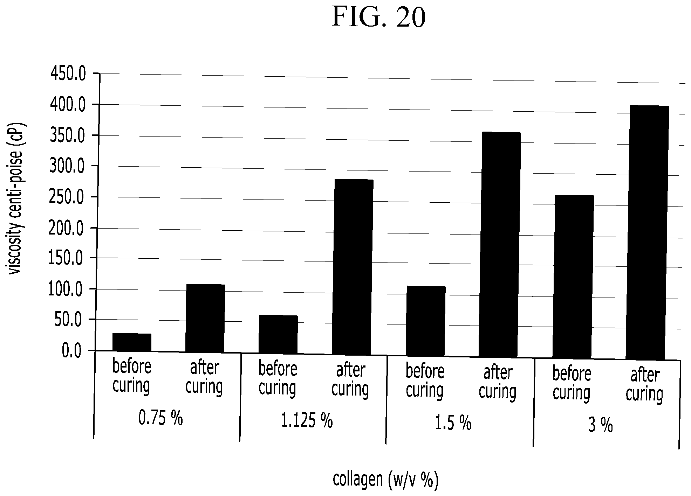

The results are shown in FIG. 20. Referring to FIG. 20, the viscosity of the gels of compositions 2 to 5 before curing was measured to be between 25 and 270 cP, and the viscosity after curing increased to 110 to 410 cP. In other words, depending on the composition, the viscosity after curing may increase by 1.5 to 4 times compared to before curing.

### Stability of collagen gel including riboflavin

To confirm the stability of the collagen gel including riboflavin, the collagen gel of composition number 4 in Table 1 was irradiated with light for 30 seconds, placed in 500 µl of purified water, and stored at 37 °C for 7, 14, and 30 days.. As a control, collagen gel with composition number 4 was placed in 500 µl of purified water without irradiation of light and stored at 37 °C for 7, 14, and 30 days.

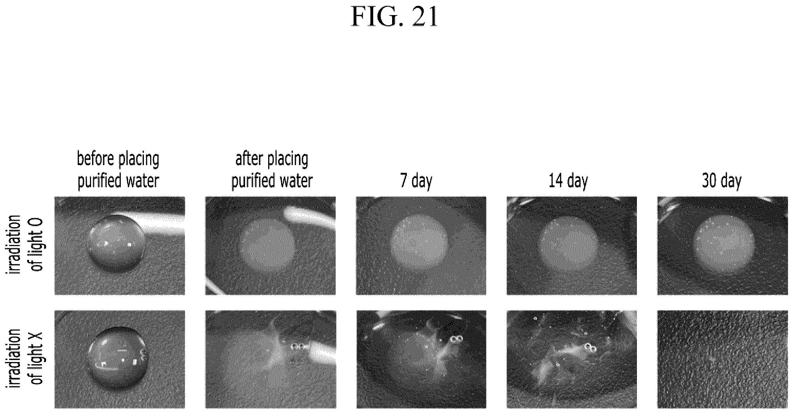

The results are illustrated in FIG. 21. Referring to FIG. 21, the gel including purified water maintained its shape after being irradiated with light for 30 seconds, while the gel not irradiated with light did not maintain its shape and was mixed with purified water. Therefore, it was confirmed that the collagen gel including riboflavin of composition number 4, which was irradiated with light for 30 seconds, stably formed a solid gel for up to 30 days.

### Adaptation of collagen gel including riboflavin as a graft material

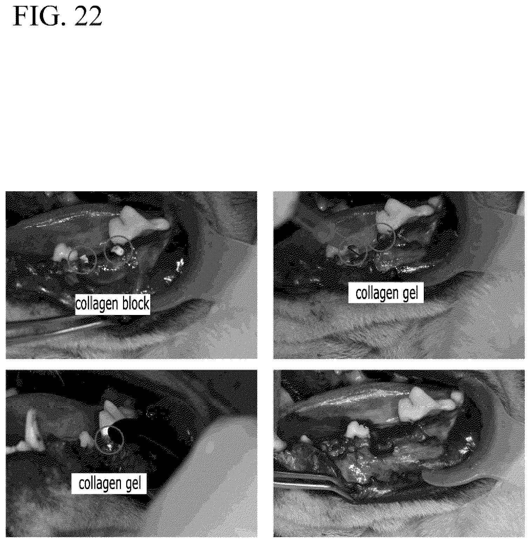

Collagen gel including riboflavin can be used in cases where fixation is required, such as collagen blocks, in local areas of the body. Collagen gel with composition number 4 in Table 1 was applied to artificially induced peri-implantitis in adult dogs. As illustrated in FIG. 22, the gums at the area where peri-implantitis appeared were incised and the area around the implant was washed with physiological saline as much as possible. After completion of washing, the collagen block including the peptide was implanted at the site of peri-implantitis (FIG. 22a). To fix the collagen block after implantation, 0.5 ml of collagen gel including riboflavin was applied (FIG. 22b), and then light was irradiated with a wavelength of 450 nm and an intensity of 2 W/cm² for 30 seconds (FIG. 22c). After confirming that the collagen gel including riboflavin had gelled, the incised gums were sutured (FIG. 22d). It was confirmed that the graft site was maintained well without any side effects even 4 weeks after implantation.

Although the experimental examples were described above, the scope of rights is not limited thereto. The embodied forms can be implemented with various modifications within the scope of the detailed description of the invention and the attached drawings, and this naturally also falls within the scope of rights.

### [Industrial Applicability]

It can be used as a graft material to treat various bone loss or defects caused by inflammation, tumor, trauma, and skeletal disease.

### [Sequence Listing Text]

SEQ ID NO.1: GKCSTRGRKS SRRKK
SEQ ID NO.2: CPRRYKQIGT CGLPGTKCCK KP
SEQ ID NO.3: GKCSTRGRKC CRRKK
SEQ ID NO.4: RKIEIVRKKP IFKKATVT
SEQ ID NO.5: CKRKKKGKGL GKKRDPCLRK YK

## Claims

1. A bioabsorbable and photocurable composition, comprising
a photosensitizer and collagen,
wherein the bioabsorbable and photocurable composition is applied onto an in vivo graft site filled with a graft material, so as to cover the graft material while filling an empty space of the graft site, and
the photosensitizer is activated by the irradiation of visible light so that the collagen is cross-linked, and the collagen cross-linked by the visible light fills the minute empty space of the graft site and closely binds the graft material to fix the graft material.

2. The bioabsorbable and photocurable composition of claim 1, wherein
a viscosity of the photocurable composition after irradiation of visible light increases by 1.5 to 4.5 times compared to before irradiation of visible light.

3. The bioabsorbable and photocurable composition of claim 1, wherein the photosensitizer is riboflavin.

4. The bioabsorbable and photocurable composition of claim 3, wherein a weight ratio of the riboflavin : collagen is 1 : 0.00056 to 1 : 0533.

5. The bioabsorbable and photocurable composition of claim 3, wherein
the visible light is a light source in a blue wavelength range and has a maximum output of 2 W/cm².

6. A bioabsorbable guided tissue regeneration composition, comprising
a graft material including a first agent including collagen as a main ingredient and a second agent including a peptide formulated to be immersed in the first agent; and
a bioabsorbable and photocurable composition including a photosensitizer and collagen, wherein the bioabsorbable and photocurable composition is applied to an graft site filled with a graft material so as to cover the graft material while filling an empty space of the graft site, and the photosensitizer is activated by the irradiation of visible light so that the collagen is cross-linked, and the collagen cross-linked by the visible light fills the fine empty space of the graft site and closely binds the graft material to fix the graft material.

7. The bioabsorbable guided tissue regeneration composition of claim 6, wherein
the peptide has antibacterial activity, anti-inflammatory activity, antibacterial activity and anti-inflammatory activity, antibacterial activity, anti-inflammatory activity, and cell-penetrating properties.

8. The bioabsorbable guided tissue regeneration composition of claim 7, wherein
the peptide is represented by any one of the amino acid sequences of SEQ ID NOs: 1 to 5.

9. The bioabsorbable guided tissue regeneration composition of claim 7, wherein
the graft material includes 10⁻⁴ to 0.5 parts by weight of the peptide.

10. The bioabsorbable guided tissue regeneration composition of claim 6, wherein
a viscosity of the photocurable composition after irradiation of visible light increases by 1.5 to 4.5 times compared to before irradiation of visible light.

11. The bioabsorbable guided tissue regeneration composition of claim 6, wherein
the photosensitizer is riboflavin.

12. The bioabsorbable guided tissue regeneration composition of claim 11, wherein
a weight ratio of the riboflavin : collagen is 1 : 0.00056 to 1 : 0.0533.

13. The bioabsorbable guided tissue regeneration composition of claim 6, wherein
the visible light is a light source in a blue wavelength range and has a maximum output of 2 W/cm².

14. A grafting method, comprising
filling a vivo graft site with a graft material;
applying a bioabsorbable and photocurable composition including a photosensitizer and collagen onto the graft site filled with the graft material, thereby filling the empty space of the graft site and covering the graft material; and
irradiating visible light to the bioabsorbable and photocurable composition to activate the photosensitizer and cross-link the collagen, so that the collagen cross-linked by the visible light fills the fine empty space of the graft site and closely binds the graft material to fix the graft material.

15. The grafting method of claim 14, wherein
before filling the graft material,
pretreating the vivo graft site is further included.

16. The grafting method of claim 15, wherein
the graft site is a periodontal disease site,
the pretreating includes scaling or scaling and root planing.

17. The grafting method of claim 14, wherein
a viscosity of the photocurable composition after irradiation of visible light increases by 1.5 to 4.5 times compared to before irradiation of visible light.

18. The grafting method of claim 14, wherein
the photosensitizer is riboflavin.

19. The grafting method of claim 18, wherein
a weight ratio of the riboflavin : collagen is 1 : 0.00056 to 1 : 0.0533.

20. The grafting method of claim 14, wherein
the visible light is a light source in a blue wavelength range and has a maximum output of 2 W/cm².

21. The grafting method of claim 14, wherein
a light irradiator that irradiates visible light penetrates into the the bioabsorbable and photosensitive composition and delivers the light source.

22. The grafting method of claim 14, wherein
a first agent including collagen as a main ingredient and a second agent including a peptide formulated to be immersed in the first agent.

23. The grafting method of claim 22, wherein
the peptide has antibacterial activity, anti-inflammatory activity, antibacterial activity and anti-inflammatory activity, antibacterial activity, anti-inflammatory activity, and cell-penetrating properties.

24. The grafting method of claim 23, wherein
the peptide is represented by any one of the amino acid sequences of SEQ ID NOs: 1 to 5.

25. The grafting method of claim 23, wherein
the graft material includes 10⁻⁴ to 0.5 parts by weight of the peptide.
